# EUROPÄISCHE PATENTANMELDUNG

(11) **EP 4 438 076 A1**
(43) Veröffentlichungstag der Anmeldung: **02.10.2024**
(21) Anmeldenummer: 23165736.2
(22) Anmeldetag: 30.03.2023
(51) Int. Cl.: A61M 5/142, A61B 5/00, A61M 5/172, G06F 1/16

(54) **ELEKTRISCHE VERBINDUNG ZWISCHEN PUMPENGEHÄUSE UND FRONTKLAPPE**

(71) Anmelder: B. Braun Melsungen AG, 34212 Melsungen (DE)
(72) Erfinder: SCHWARZ, Jan, 34212 Melsungen (DE); ERLEN, Christoph, 34132 Kassel (DE); NIEMEIER, Carsten, 34130 Kassel (DE); BATZDORF, Stefan, 36041 Fulda (DE); KAUBA, Michael, 34277 Fuldabrück (DE); OSTERMOELLER, Michael, 36251 Bad Hersfeld (DE); HOEVEL, Stephan, 34121 Kassel (DE); RICHARDT, Mario, 34289 Zierenberg (DE)
(74) Vertreter: Winter, Brandl - Partnerschaft mbB

(57) **Zusammenfassung**

Die vorliegende Offenbarung betrifft eine medizinische Pumpe (1) mit einem quaderförmigen Pumpengehäuse (2) und einer schwenkbar an dem Pumpengehäuse (2) angelenkten Frontklappe (3), in welcher ein Display (4), insbesondere ein Touch-Display, und/oder Bedienelemente vorgesehen sind, wobei die Frontklappe (3) mit zumindest einem ersten und zumindest einem zweiten Scharnierelement (5) an dem Pumpengehäuse (2) angebracht ist, wobei das zumindest eine erste und/oder das zumindest eine zweite Scharnierelement (5) derart vorgesehen und ausgebildet ist, um zumindest eine elektrische Verbindung (6) zwischen dem Pumpengehäuse (2) und der Frontklappe (3) zur elektrischen Anbindung und zum Datenaustausch aufzuweisen und/oder zu bilden

## Beschreibung

### Technisches Gebiet

Die vorliegende Offenbarung betrifft eine medizinische Pumpe, insbesondere Fluidpumpe und deren elektrische Verbindung zwischen Pumpengehäuse und Frontplatte.

### Hintergrund der Offenbarung

In der Medizin werden verbreitet Fluidpumpen, insbesondere Spritzenpumpen und Schlauchpumpen zur Versorgung eines Patienten mit einer definierten Dosis an Medikamenten eingesetzt. Da es sich bei solchen Fluidpumpen um kostspielige medizinische Ausrüstung handelt, ist im Regelfall nicht jeder Patientenplatz mit einer solchen medizinischen Pumpe ausgestattet, sondern es wird im Bedarfsfall die medizinische Pumpe an dem Patientenplatz installiert. Aus diesem Grund handelt es sich bei solchen medizinischen Pumpen häufig um mobile bzw. teilmobile Vorrichtungen, die von einem Behandler zu dem betreffenden Patienten bzw. Patientenplatz verbracht und dort installiert werden.

Bekannte Pumpen weisen Touch-Displays bzw. Bildschirme und Tasten auf, auf denen Informationen ausgegeben werden und Benutzer Anweisungen an die Pumpe eingeben können. So kann beispielsweise das richtige Medikament und eine Dosierung eingegeben werden. Bei einer Spritzenpumpe kann das Modell der Spritze und die Dosierung des Medikaments eingegeben werden. Hierbei besteht die Notwendigkeit einer elektrische Verbindung zur Spannungsversorgung und Datenübertragung zwischen einem Pumpengehäuse und dem Display in der Frontklappe zur Verfügung zu stellen.

Für eine solche elektrische Verbindung ist aus dem Stand der Technik die Verwendung eines Displaykabels, insbesondere eines Flachbandkabels, bekannt. Ein Flachbandkabel hat den Nachteil, dass es entweder aufgrund der schwenkbaren Frontklappe immer wieder geknickt wird. Darüber hinaus ist die Verwendung eines Displaykabels für Verschmutzung prädestiniert.

Daher liegt der vorliegenden Offenbarung die Aufgabe zugrunde, die Nachteile aus dem Stand der Technik zu beheben oder zumindest zu mindern. Ferner ist es Ziel eine elektrische Verbindung zwischen dem Pumpengehäuse und der Frontklappe bereitzustellen, welche sowohl kostengünstig als auch robust ausgebildet ist.

Die Aufgabe werden hinsichtlich einer gattungsgemäßen medizinische Pumpe offenbarungsgemäß durch den Gegenstand des Anspruchs 1 gelöst.

Die medizinische Pumpe hat ein quaderförmiges Pumpengehäuse und eine schwenkbar an dem Pumpengehäuse angelenkte Frontklappe, in welcher ein Display, insbesondere ein Touch-Display, und/oder Bedienelemente vorgesehen sind. Die Frontklappe ist mit zumindest einem ersten und zumindest einem zweiten Scharnierelement an dem Pumpengehäuse angebracht. Hierbei ist das zumindest eine erste und/oder das zumindest eine zweite Scharnierelement derart vorgesehen und ausgebildet ist, um zumindest eine elektrische Verbindung zwischen dem Pumpengehäuse und der Frontklappe zur elektrischen Anbindung, Versorgung und zum Datenaustausch aufzuweisen und/oder zu bilden .

In anderen Worten hat die medizinische Pumpe ein Pumpengehäuse, an dessen Vorderseite eine schwenkbar gelagerte Frontklappe, in welche ein Display/ eine Anzeige und/ oder Bedienelemente beherbergt ist. Die Frontklappe ist mittels zwei oder mehreren Scharnierelementen/ Scharnieren an dem Pumpengehäuse gelagert. Das eine oder die mehreren Scharnierelemente realisieren jeweils eine elektrische Verbindung zwischen dem Pumpengehäuse und der Frontklappe.

Dadurch wird das Problem der elektrischen Anbindung der Frontklappe zu dem Pumpengehäuse bezüglich Energie, Daten und Masse gelöst und gleichzeitig kann eine Verschmutzung vermieden werden, welche mit einem Flachbandkabel einhergeht. Darüber hinaus können durch diese Lösung die Herstellerkosten gegenüber dem Stand der Technik gesenkt werden. Durch den Effekt, dass kein Kabel mehr beim Auf und Zu Schwenken der Frontklappe geknickt wird, wird auch die Robustheit und Lebensdauer erhöht. Ferner ist die Art der Bereitstellung einer elektrischen Verbindung weniger anfällig für Störungen, beispielsweise in Bezug auf die elektromagnetische Verträglichkeit.

Weitere vorteilhafte Aspekte der vorliegenden Offenbarung werden nachstehend gemäß den Unteransprüchen beschrieben.

Es ist bevorzugt, wenn das zumindest eine erste und/oder das zumindest eine zweite Scharnierelement einem Massepotential ausgesetzt ist. Das heißt, dass das eine oder die mehreren Scharnierelemente dem Erdpotential ausgesetzt sind.

Es ist von Vorteil, wenn das zumindest eine erste und/oder das zumindest eine zweite Scharnierelement dazu vorgesehen und ausgebildet ist, um auf der Seite des Pumpengehäuses mit einer ersten internen Leiterplatine des Pumpengehäuses und auf der Seite der Frontklappe mit einer zweiten internen Leiterplatine der Frontklappe elektrisch verbunden zu sein.

Ferner ist es bevorzugt, wenn das zumindest eine erste und/oder zweite Scharnierelement zum Austausch elektrischer Potentiale vorgesehen und ausgebildet ist.

Hierbei ist es vorteilhaft, wenn das zumindest eine erste oder zweite Scharnierelement dazu vorgesehen und ausgebildet ist, um elektrische Massepotentiale zu übertragen.

Darüber hinaus ist es bevorzugt, wenn das zumindest andere erste und/oder zweite Scharnierelement dazu vorgesehen und ausgebildet ist, um zumindest eine Versorgungsspannung und/oder zumindest ein Datensignal zu übertragen.

In anderen Worten ist es beliebig kombinierbar, ob ein erstes Scharnierelement das elektrische Massepotential und ein zweites Scharnierelement die Versorgungsspannung übertragt oder, ob ein erstes Scharnierelement das elektrische Massepotential und ein zweites Scharnierelement das Datensignal und die Versorgungsspannung überträgt. Alternativ kann auch für sowohl das elektrische Massepotential als auch die Versorgungsspannung als auch das Datensignal jeweils ein eigenes Scharnierelement vorgesehen sein.

Das bedeutet, dass die elektrischen Verbindungen zum Austausch elektrischer Potentiale dienen und hierbei ein oder mehrere Scharniere für die Übertragung des elektrischen Massepotentials verwendet werden und zusätzlich ein anderes oder mehrere andere Scharniere für die Übertragung einer oder mehrerer Versorgungsspannungen verwendet werden. Hierbei ist eine kombinierte Übertragung von unterschiedlichen Signalen pro Scharnier denkbar, bspw. von Massepotential und Versorgungsspannung oder Masse und Datensignale.

Es ist von Vorteil, wenn das Datensignal mittels Modulation, insbesondere mittels Pulsweitenmodulation oder mittels Aufprägung eines höher frequenten Kleinsignals oder mittels Frequenzmultiplexverfahren, auf der Versorgungsspannung oder einem Trägersignal übertragbar ist. Frequenzmultiplexverfahren (engl. frequency-division multiplexing, FDM oder frequency-division multiple access, FDMA) ist ein nachrichtentechnisches Multiplexverfahren, mit dem gleichzeitig mehrere Signale auf mehrere Träger verteilt übertragen werden können. Die Träger sind mehreren unterschiedlichen Frequenzen zugeordnet, weswegen auch der Begriff Frequenzmultiplex verwendet wird.

In anderen Worten dient die elektrische Verbindung der Übertragung von Informationen und Daten. Hierbei ist es vorgesehen, wenn ein weiteres oder weitere Scharniere für die Übertragung eines oder mehrerer Datensignale verwendet werden. Der Datenaustausch ist unter anderem per Modulation auf zum Beispiel die Versorgungsspannung oder auf einem anderen Trägersignal vorgesehen. Dies hat den Vorteil, dass das Verbauen von zwei Scharnieren ausreichend ist.

Es ist bevorzugt, wenn in dem Pumpengehäuse eine Steuereinheit angeordnet ist, welche dazu vorgesehen und ausgebildet ist, um mit der Frontklappe zu kommunizieren.

In anderen Worten bedeutet das, dass es von Vorteil ist, wenn der Datenaustausch zur Kopplung der Anzeige und/ oder der Bedienelemente in der Frontklappe mit einer Steuereinheit im Pumpengehäuse verwendet wird. Dies hat den Vorteil, dass das Flachbandkabel zwischen der Frontklappe und dem Pumpengehäuse aus dem Stand der Technik entfällt. Zudem sind Signalleitungen auf diese Weise vor Störungen von außen geschützt.

Es ist vorteilhaft, wenn das zumindest eine erste und/ oder das zumindest eine zweite Scharnierelement mit einem Scharnierbolzen und einer Scharnierbolzenhülse ausgebildet ist, wobei der Scharnierbolzen innerhalb der Scharnierbolzenhülse auf der Schwenkachse geführt ist.

Vorzugsweise ist der Scharnierbolzen geerdet.

Es ist von Vorteil, wenn durch das zumindest eine erste und/oder das zumindest eine zweite Scharnierelement, vorzugsweise durch den Scharnierbolzen, zumindest ein elektrischer Leiter geführt ist, welcher auf der Seite des Pumpengehäuses mit einer internen Leiterplatine des Pumpengehäuses und/oder auf der Seite der Frontklappe mit einer internen Leiterplatine der Frontklappe elektrisch verbunden ist.

Es ist bevorzugt, wenn das zumindest eine erste und/ oder das zumindest eine zweite Scharnierelement, vorzugsweise der Scharnierbolzen, mittels zumindest einer elektrisch leitenden Feder mit der internen Leiterplatine des Pumpengehäuses und/ oder mittels zumindest einer weiteren elektrisch leitenden Feder mit der internen Leiterplatine der Frontklappe elektrisch verbunden ist.

Bevorzugter Weise ist der Scharnierbolzen mit der elektrisch leitenden Feder verbunden, wobei die Feder auf eine Massefläche der internen Leiterplatine drückt.

Es ist vorteilhaft, wenn das zumindest eine erste und/ oder das zumindest eine zweite Scharnierelement, vorzugsweise der Scharnierbolzen, mittels zumindest einer elektrisch leitenden Feder mit der internen Leiterplatine des Pumpengehäuses elektrisch verbunden ist und durch das zumindest eine erste und/oder das zumindest eine zweite Scharnierelement, vorzugsweise durch den Scharnierbolzen, zumindest ein elektrischer Leiter geführt ist, welcher auf der Seite der Frontklappe mit einer internen Leiterplatine der Frontklappe elektrisch verbunden ist.

In anderen Worten kann die elektrische Verbindung/ Anbindung der Scharnierachse bzw. des Scharnierbolzens an die erste und/oder zweite interne elektrische Leiterplatine der Frontklappe bzw. des Hauptkörpers wie folgt realisiert werden. Die elektrische Verbindung/ Anbindung kann mittels einer elektrisch leitenden Feder, die auf die Achse bzw. den Scharnierbolzen wirkt, erfolgen. Alternativ oder zusätzlich kann die elektrische Verbindung/ Anbindung durch den elektrischen Leiter erfolgen, der innerhalb des Scharnierbolzen auf der Scharnierachse/ Schwenkachse verlaufend vorgesehen und ausgebildet ist. Ferne kann ein durch den Scharnierbolzen geführter elektrischer Leiter beiderseits an die erste und zweite interne Leiterplatine der Frontklappe bzw. des Pumpengehäuses angeschlossen sein. Mögliche Realisierung sind optional in symmetrischer Form durch beide Achsen der Scharniere vorgesehen.

### Kurzbeschreibung der Figuren

Die Offenbarung wird nachfolgend anhand eines bevorzugten Ausführungsbeispiels mit Hilfe von Figuren näher erläutert.
Fig. 1 ist eine Perspektivansicht einer offenbarungsgemäßen, medizinischen Pumpe gemäß dem bevorzugten Ausführungsbeispiel;
Fig. 2 ist eine Darstellung zur Veranschaulichung einer Frontklappe mit einer Rückschale gemäß dem bevorzugten Ausführungsbeispiel;
Fig. 3 ist eine Schnittansicht der medizinischen Pumpe in Längsrichtung orthogonal zur Frontklappenlängsrichtung gemäß dem Stand der Technik;
Fig. 4 und Fig. 5 zeigen eine vergrößerte Schnittansicht einer elektrischen Verbindung zwischen dem Pumpengehäuse und der Frontklappe der medizinischen Pumpe gemäß einem bevorzugten Ausführungsbeispiel;
Fig. 6 zeigt eine Scharnierplatte angebracht an der Gehäuseunterschale der medizinischen Pumpe gemäß einem bevorzugten Ausführungsbeispiel;
Fig. 7, Fig. 8 und Fig. 9 zeigen eine elektrische Verbindung der medizinischen Pumpe gemäß einem weiteren bevorzugten Ausführungsbeispiel.

### Beschreibung der Ausführungsbeispiele

Nachstehend werden Ausführungsbeispiele der vorliegenden Offenbarung auf der Basis der zugehörigen Figuren beschrieben.

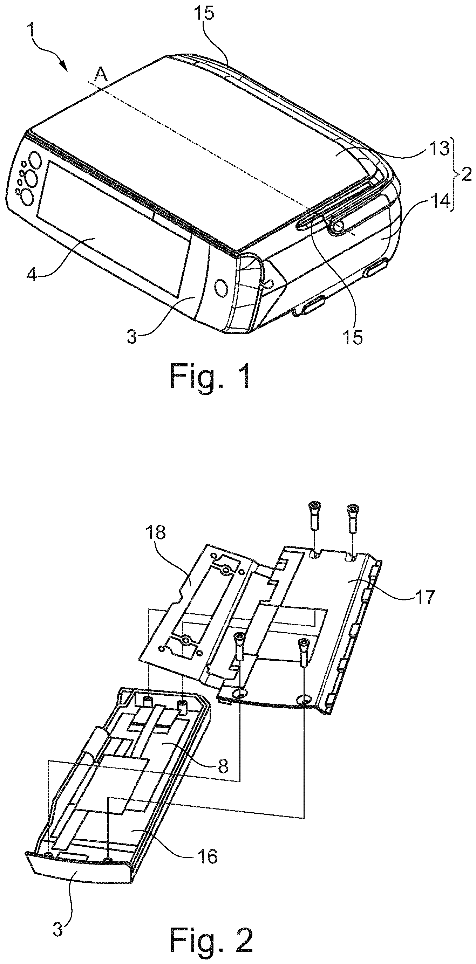

In Fig. 1 ist eine medizinische Pumpe 1 gemäß dem bevorzugten Ausführungsbeispiel gezeigt. Dabei ist die in Fig. 2 gezeigte medizinische Pumpe 1 als Infusionspumpe ausgeführt. Selbstverständlich kann die offenbarungsgemäße medizinische Pumpe 1 auch als Spritzenpumpe ausgebildet sein. Wie in Fig. 1 gezeigt, weist die medizinische Pumpe 1 ein im Wesentlichen quaderförmiges Pumpengehäuse 2 auf, an dessen Vorderseite eine Frontklappe 3 schwenkbar angelenkt ist. Auf einer Vorderseite der Frontklappe 3 sind mehrere Bedientasten, mehrere Signalleuchten und ein Display 4 angebracht.

Das Pumpengehäuse 2 weist ferner eine Gehäuseoberschale/ -oberseite 13 und eine Gehäuseunterschale/ -unterseite 14 auf, welche vorzugsweise lösbar miteinander verbunden werden können und so das Pumpengehäuse 2 ausbilden. An der Gehäuseoberschale 13 ist ein als U-förmiger Griffbügel ausgeführter Tragegriff 15 schwenkbar angelenkt. Dabei umgreift der Tragegriff 15 die Gehäuseoberschale 13 in einer Breitenrichtung der medizinischen Pumpe 1 bzw. eine Frontklappenlängsrichtung.

Fig. 2 zeigt eine Rückseite der Frontklappe 3 mit einer montierten Bedieneinheit 16, welche rückseitig dargestellt ist. Eine Rückwand 17 wird auf die Frontklappe 3 der Bedieneinheit 16 aufgesetzt. Die Rückwand 17 ist im Wesentlichen eine flache Platte und wird mit der Frontklappe 3 verschraubt. Die Rückwand 17 schützt die interne Leiterplatine 8 bzw. die Elektronik in der Frontklappe 3 bzw. der Bedieneinheit 16. An der Rückwand 17 ist eine metallene Scharnierplatte 18 befestigt. Die Scharnierplatte 18 ist klappbar mit der Rückwand 17 verbunden. Das heißt die Scharnierplatte 18 kann gegen die Rückwand 17 verschwenkt werden. Die Scharnierplatte 18 ist mit dem Pumpengehäuse 2 verbunden. Also ist die Frontklappe 3 durch die Scharnierplatte 18 schwenkbar mit dem Pumpengehäuse 2 verbunden.

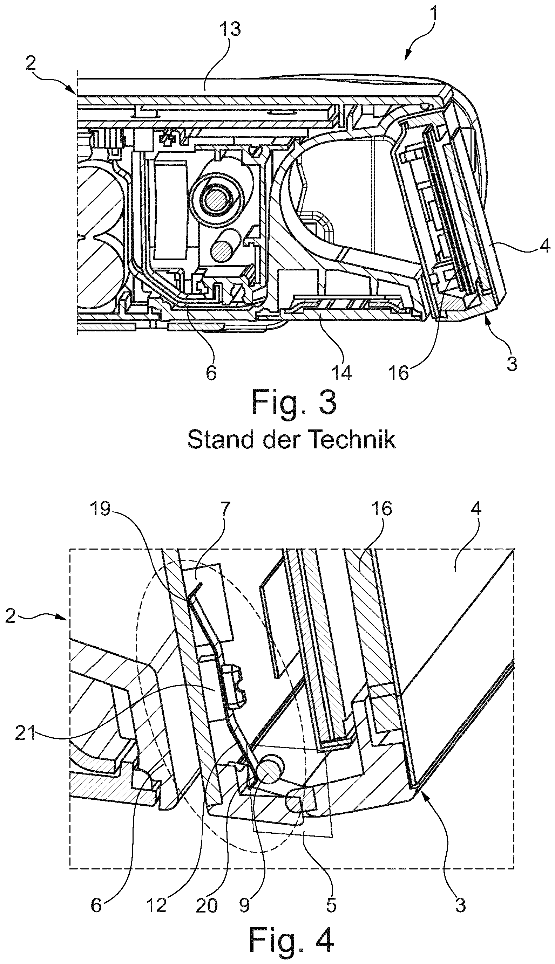

Fig. 3 ist eine Schnittansicht der medizinischen Pumpe 2, insbesondere einer Spritzenpumpe, in Längsrichtung orthogonal zur Frontklappenlängsrichtung gemäß dem Stand der Technik. Der Aufbau für die in Fig. 1 gezeigte Infusionspumpe ist äquivalent. Fig. 3 zeigt die medizinische Pumpe 1 mit dem Pumpengehäuse 2 und der Frontklappe 3. In der Frontklappe 3 ist die Bedieneinheit 16 und das Display 4 dargestellt. Ferner ist in Fig. 3 eine elektrische Verbindung 6 gemäß dem Stand der Technik gezeigt. Die elektrische Verbindung ist in dem Fall ein Displaykabel, welches die Elektronik in dem Pumpengehäuse 2 mit der Elektronik in der Frontklappe elektrisch verbindet. Gemäß der vorliegenden Offenbarung gilt es das Displaykabel zu ersetzen bzw. zu vermeiden.

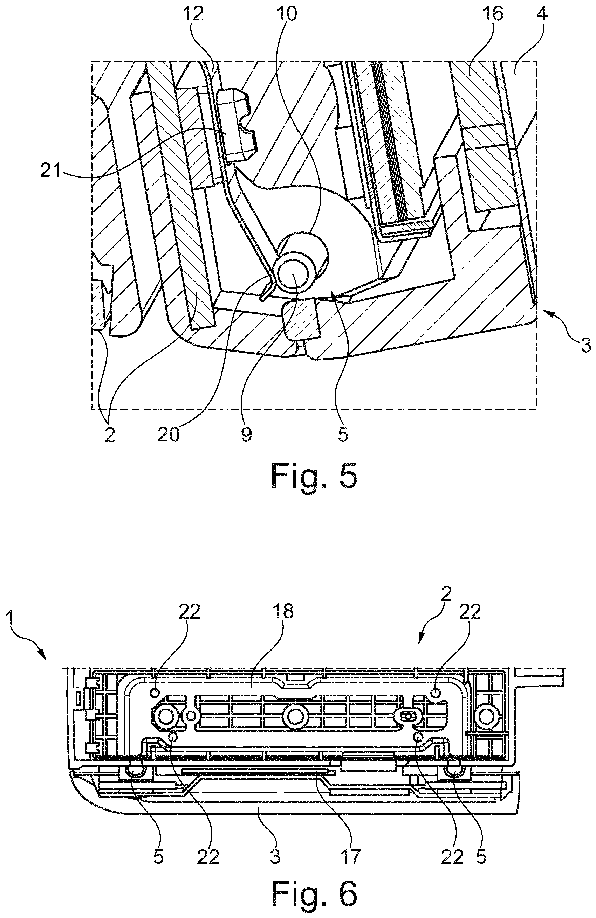

Fig. 4 und Fig. 5 zeigen eine vergrößerte Schnittansicht einer elektrischen Verbindung 6 zwischen dem Pumpengehäuse 2 und der Frontklappe 3 der medizinischen Pumpe 1 gemäß einem bevorzugten Ausführungsbeispiel. In Fig. 4 und Fig. 5 ist ein erstes Scharnierelement 5 derart vorgesehen und ausgebildet, um zumindest eine elektrische Verbindung 6 zwischen dem Pumpengehäuse 2 und der Frontklappe 3 zur elektrischen Versorgung und zum Datenaustausch aufzuweisen, dargestellt. Das Scharnierelement 5 ist dazu vorgesehen und ausgebildet, um auf der Seite des Pumpengehäuses 2 mit einer ersten internen Leiterplatine 7 des Pumpengehäuses 2 und auf der Seite der Frontklappe 3 mit einer zweiten internen Leiterplatine 8 der Frontklappe 3 elektrisch verbunden zu sein. Die zweite interne Leiterplatine 8 ist in Fig. 2 zu sehen und die Kontaktierung (nicht gezeigt) der zweiten internen Leiterplatine 8 kann gemäß der abgebildeten Feder 12 ausgebildet sein. Das Scharnierelement 5 mit einem Scharnierbolzen 9 und einer Scharnierbolzenhülse 10 (siehe Fig. 5) ausgebildet ist, wobei der Scharnierbolzen 9 innerhalb der Scharnierbolzenhülse 10 auf einer Schwenkachse geführt ist.

Das Scharnierelement 5, hier der Scharnierbolzen 9, ist mittels zumindest einer elektrisch leitenden Feder 12 mit der ersten internen Leiterplatine 7 des Pumpengehäuses 2 verbunden. Hierbei drückt ein erstes Ende 19 der elektrisch leitenden Feder 12 auf die interne Leiterplatine 7 und ein zweites Ende 20 kontaktiert den Scharnierbolzen 9, wodurch eine elektrische Verbindung 6 bereitgestellt wird. Das heißt, der Scharnierbolzen 9 ist derart ausgebildet, um elektrische Potentiale übertragen zu können. Die elektrisch leitende Feder 12 ist mit einer Federbefestigung 21 durch Klemmen oder Schrauben an das Pumpengehäuse 2 angebracht.

Fig. 6 zeigt eine Scharnierplatte 18 angebracht an der Gehäuseunterschale 14 der medizinischen Pumpe 1 gemäß einem bevorzugten Ausführungsbeispiel. Hierbei ist die Scharnierplatte 18 mittels vier Befestigungsmittel an einer Unterseite der Gehäuseunterschale 14 des Pumpengehäuses 2 angebracht. Am Übergang der Scharnierplatte 18 zu der Rückwand 17 der Frontklappe 3 sind ein erstes und ein zweites Scharnierelement 5 gezeigt, welche als elektrische Verbindung ausgebildet sind.

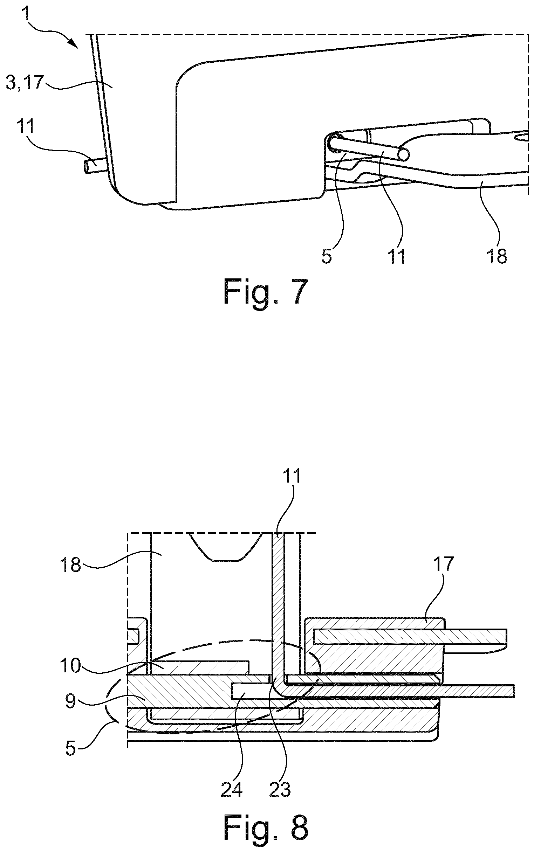

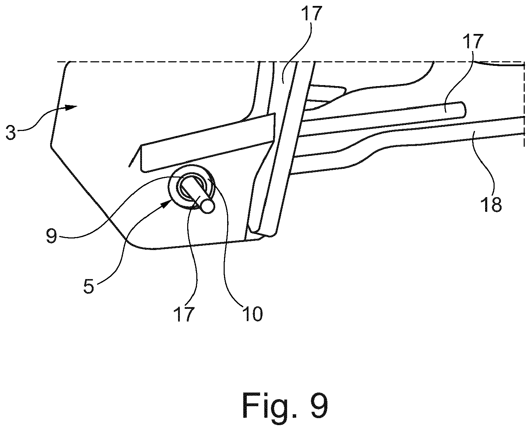

Fig. 7, Fig. 8 und Fig. 9 zeigen eine elektrische Verbindung 6 der medizinischen Pumpe 1 gemäß einem weiteren bevorzugten Ausführungsbeispiel. In den Fign. 7, 8 und 9 ist die an die Frontplatte 3 angebrachte Rückwand 17 und die über das Scharnierelement 5 damit verbundene Scharnierplatte 18, welche an der Gehäuseunterschale 14 des Pumpengehäuses 2 angebracht ist. Gemäß diesem Ausführungsbeispiel führt ein elektrischer Leiter 11 durch das Scharnierelement 5 um eine elektrische Verbindung zwischen der Frontplatte 3 und dem Pumpengehäuse 2 zur Verfügung zu stellen.

In Fig. 8 ist gezeigt, dass der elektrische Leiter 11 von der Rückwand 18 durch eine Scharnierbolzenöffnung 23 in einen Hohlraum 24 des Scharnierbolzens 9 geführt ist. In anderen Worten ist der Scharnierbolzen 9 zumindest teilweise hohl, um einen elektrischen Leiter 11 über eine in dem Scharnierbolzen 9 befindliche Scharnierbolzenöffnung 23 aufzunehmen. Der elektrische Leiter 11 und die Scharnierbolzenöffnung 23 ist derart in den Hohlraum 24 des Scharnierbolzens 9 eingeführt, dass auch bei der Rotations- bzw. Schwenkbewegung der elektrische Leiter 11 nicht geknickt wird. Somit ist ein Ende des elektrischen Leiters 11 mit der ersten internen Leiterplatine 7 und ein anderes Ende des elektrischen Leiters 11 mit der zweiten internen Leiterplatine 8 elektrisch verbunden.

### Bezugszeichen

- 1: medizinische Pumpe
- 2: Pumpengehäuse
- 3: Frontklappe
- 4: Display
- 5: Scharnierelement
- 6: elektrische Verbindung
- 7: erste interne Leiterplatine
- 8: zweite interne Leiterplatine
- 9: Scharnierbolzen
- 10: Scharnierbolzenhülse
- 11: Elektrischer Leiter
- 12: Feder
- 13: Gehäuseoberschale
- 14: Gehäuseunterschale
- 15: Tragegriff
- 16: Bedieneinheit
- 17: Rückwand
- 18: Scharnierplatte
- 19: erstes Ende
- 20: zweites Ende
- 21: Federbefestigung
- 22: Befestigungsmittel
- 23: Scharnierbolzenöffnung
- 24: Hohlraum

## Patentansprüche

1. Medizinische Pumpe (1) mit einem quaderförmigen Pumpengehäuse (2) und einer schwenkbar an dem Pumpengehäuse (2) angelenkten Frontklappe (3), in welcher ein Display (4), insbesondere ein Touch-Display, und/oder Bedienelemente vorgesehen sind, wobei die Frontklappe (3) mit zumindest einem ersten und zumindest einem zweiten Scharnierelement (5) an dem Pumpengehäuse (2) angebracht ist,
**dadurch gekennzeichnet, dass**
das zumindest eine erste und/oder das zumindest eine zweite Scharnierelement (5) derart vorgesehen und ausgebildet ist, um zumindest eine elektrische Verbindung (6) zwischen dem Pumpengehäuse (2) und der Frontklappe (3) zur elektrischen Anbindung, Versorgung und zum Datenaustausch aufzuweisen und/oder zu bilden.

2. Medizinische Pumpe (1) gemäß Anspruch 1, **dadurch gekennzeichnet, dass** das zumindest eine erste und/oder das zumindest eine zweite Scharnierelement (5) dazu vorgesehen und ausgebildet ist, um auf der Seite des Pumpengehäuses (2) mit einer ersten internen Leiterplatine (7) des Pumpengehäuses (2) und auf der Seite der Frontklappe (3) mit einer zweiten internen Leiterplatine (8) der Frontklappe (3) elektrisch verbunden zu sein.

3. Medizinische Pumpe (1) gemäß Anspruch 2, **dadurch gekennzeichnet, dass** das zumindest eine erste und/oder zweite Scharnierelement (5) zum Austausch elektrischer Potentiale vorgesehen und ausgebildet ist.

4. Medizinische Pumpe (1) gemäß Anspruch 2 oder 3, **dadurch gekennzeichnet, dass** das zumindest eine erste oder zweite Scharnierelement (5) dazu vorgesehen und ausgebildet ist, um elektrische Massepotentiale zu übertragen.

5. Medizinische Pumpe (1) gemäß Anspruch 4, **dadurch gekennzeichnet, dass** das zumindest andere erste und/oder zweite Scharnierelement (5) dazu vorgesehen und ausgebildet ist, um zumindest eine Versorgungsspannung und/oder zumindest ein Datensignal zu übertragen.

6. Medizinische Pumpe (1) gemäß Anspruch 5, **dadurch gekennzeichnet, dass** das Datensignal mittels Modulation, insbesondere mittels Pulsweitenmodulation oder mittels Aufprägung eines höher frequenten Kleinsignals oder mittels Frequenzmultiplexverfahren, auf der Versorgungsspannung oder einem Trägersignal übertragbar ist.

7. Medizinische Pumpe (1) gemäß einem der Ansprüche 2 bis 6, **dadurch gekennzeichnet, dass** das zumindest eine erste und/ oder das zumindest eine zweite Scharnierelement (5) mit einem Scharnierbolzen (9) und einer Scharnierbolzenhülse (10) ausgebildet ist, wobei der Scharnierbolzen (9) innerhalb der Scharnierbolzenhülse (10) auf einer Schwenkachse geführt ist.

8. Medizinische Pumpe (1) gemäß einem der Ansprüche 7, **dadurch gekennzeichnet, dass** durch das zumindest eine erste und/oder das zumindest eine zweite Scharnierelement (5), vorzugsweise durch den Scharnierbolzen (9), zumindest ein elektrischer Leiter (11) geführt ist, welcher auf der Seite des Pumpengehäuses (2) mit einer internen Leiterplatine (7) des Pumpengehäuses (2) und/oder auf der Seite der Frontklappe (3) mit einer internen Leiterplatine (8) der Frontklappe (3) elektrisch verbunden ist.

9. Medizinische Pumpe (1) gemäß Anspruch 7, **dadurch gekennzeichnet, dass** das zumindest eine erste und/ oder das zumindest eine zweite Scharnierelement (5), vorzugsweise der Scharnierbolzen (9), mittels zumindest einer elektrisch leitenden Feder (12) mit der ersten internen Leiterplatine (7) des Pumpengehäuses (2) und/ oder mittels zumindest einer weiteren elektrisch leitenden Feder (12) mit der zweiten internen Leiterplatine (8) der Frontklappe (3) elektrisch verbunden ist.

10. Medizinische Pumpe (1) gemäß Anspruch 7, **dadurch gekennzeichnet, dass** das zumindest eine erste und/ oder das zumindest eine zweite Scharnierelement (5), vorzugsweise der Scharnierbolzen (9), mittels zumindest einer elektrisch leitenden Feder (12) mit der ersten internen Leiterplatine (7) des Pumpengehäuses (2) elektrisch verbunden ist und durch das zumindest eine erste und/oder das zumindest eine zweite Scharnierelement (5), vorzugsweise durch den Scharnierbolzen (10), zumindest ein elektrischer Leiter (11) geführt ist, welcher auf der Seite der Frontklappe (3) mit der zweiten internen Leiterplatine (8) der Frontklappe (3) elektrisch verbunden ist.
